# EUROPÄISCHE PATENTANMELDUNG

(11) **EP 0 945 441 A2**
(43) Veröffentlichungstag der Anmeldung: **29.09.1999**
(21) Anmeldenummer: 99105642.5
(22) Anmeldetag: 19.03.1999
(51) Int. Cl.: C07D 235/02, C07D 233/32, C07D 233/70

(54) **Verfahren zur Herstellung von cyclischen 4-Oxoamidinen**

(30) Priorität: 27.03.1998 DE 19813435
(71) Anmelder: Degussa-Hüls Aktiengesellschaft, 60287 Frankfurt am Main (DE)
(72) Erfinder: Drauz, Karlheinz Prof. Dr., 63579 Freigericht (DE); Hoffmann, Rolf Dr., 2930 Brasschaat (BE); Pelgrims, Ivan, 2550 Kontich (BE)

(57) **Zusammenfassung**

Die vorliegende Erfindung betrifft ein Verfahren zur Herstellung von cyclischen 4-Oxoamidinen. Derartige Substanzen bilden Vorstufen zu bioaktiven Wirkstoffen. Das erfindungsgemäße Verfahren bildet einen weiteren Zugang zu dieser Verbindungsklasse.

Dadurch, daß Kondensationsprodukte von Aldehyden und Aminonitrilen oder Aminosäureamiden mit Oxidationsmitteln zur Reaktion gebracht werden, gelangt man erfindungsgemäß in guten bis sehr guten Ausbeuten zu den gewünschten cyclischen 4-Oxoamidinen.

## Beschreibung

Die vorliegende Erfindung betrifft ein Verfahren zur Herstellung von cyclischen 4-Oxoamidinen der allgemeinen Formel I.

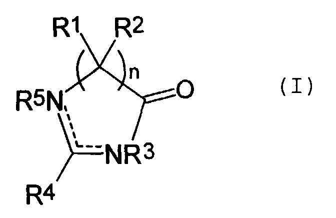

Verbindungen dieser Art sind wichtige Zwischenprodukte für die Synthese bioaktiver Wirkstoffe, wie z. B. Blutdrucksenker oder Pflanzenschutzmittel.

Bisher wurden Verbindungen der allgemeinen Formel I aus acylierten α-Aminonitrilen durch Hydrolyse der Nitrilgruppe zur Carbamoylgruppe und anschließende basenkatalysierte Cyclisierung erhalten (US 5,424,450).

In der kürzlich erschienenen Offenlegungsschrift EP 0 789 019 wird das entsprechende acylierte α-Aminonitrile in Gegenwart eines Alkohols unter sauren Bedingungen sofort zum entsprechenden 4-Oxoimidazoliniumsalz cyclisiert.

Aufgabe der vorliegenden Erfindung ist die Angabe eines weiteren Verfahrens zur Herstellung der Verbindungen der allgemeinen Formel I.

Dadurch, daß man Amidine der allgemeinen Formel I 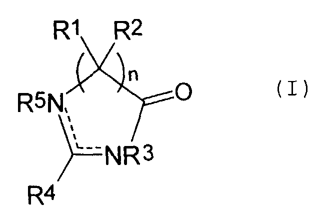 oder deren Salze
worin
R¹, R² für gleiche oder unterschiedliche Indices n unabhängig voneinander stehen für
   H, (C₁-C₈)-Alkyl, (C₂-C₈)-Alkenyl, (C₂-C₈)-Alkinyl, (C₁-C₈)-Alkoxy, (C₂-C₈)-Alkoxyalkyl, welche gegebenenfalls linear oder verzweigt vorliegen sowie einfach oder mehrfach mit Halogenen, N-, O-, P-atomhaltigen Resten substituiert sein können,
   (C₃-C₇)-Cycloalkyl, welche gesättigt und/oder ungesättigt sowie einfach oder mehrfach mit linear oder verzweigten (C₁-C₈)-Alkyl, (C₂-C₈)-Alkenyl, (C₁-C₈)-Alkoxy, (C₂-C₈)-Alkoxyalkyl, Halogenen, N-, O-, P-atomhaltigen Resten substituiert sein oder im Ring Heteroatome wie N, O, P enthalten können,
   Aryl, wie Phenyl und/oder Naphthyl, Aralkyl, wie Benzyl und/oder Phenethyl,
      Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die eben genannten Cyclen gegebenenfalls einfach oder mehrfach mit linear oder verzweigten (C₁-C₈)-Alkyl, (C₂-C₈)-Alkenyl, (C₁-C₈)-Alkoxy, (C₂-C₈)-Alkoxyalkyl, Halogenen, N-, O-, P-atomhaltigen Resten substituiert sein können
      oder
R¹ und R² über das verbindende Kohlenstoffatom zu einem 3- bis 7-gliedrigen carbocyclischen oder heterocyclischen, gesättigten oder ungesättigten Ring verbunden sein können,
   welcher gegebenenfalls einfach oder mehrfach mit linear oder verzweigten (C₁-C₈)-Alkyl, (C₂-C₈)-Alkenyl, (C₁-C₈)-Alkoxy, (C₂-C₈)-Alkoxyalkyl, Halogenen, N-, O-, P-atomhaltigen Resten substituiert sein kann,
R³, R⁴, R⁵ unabhängig voneinander die Bedeutung der Reste für R¹ annehmen können, mit der Maßgabe, daß R³ und R⁵ nicht gleichzeitig ungleich H sein dürfen und die Reste der Gruppe R³, R⁴ oder R⁵ untereinander nicht zu einem Cyclus verbunden sind,
n eine ganze Zahl zwischen 1 und 3 ist,
durch Oxidation von Cyclen der allgemeinen Formel II 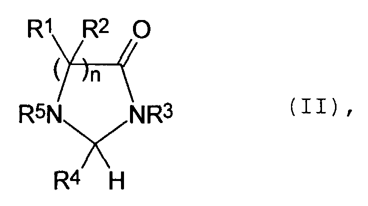 in der die Reste R¹ bis R⁵ und n die oben angegebene Bedeutung besitzen,
herstellt, gelangt man vorteilhafterweise in guten bis sehr guten Ausbeuten zu außerordentlich reinen Verbindungen der allgemeinen Formel I.

Die Verbindungen der allgemeinen Formel II lassen sich nach an sich bekannten Methoden herstellen (Journal für prakt. Chemie Band 32b, Heft 2, 1984, S. 279-86; Bull. Chem. Soc. Jpn., 1971, 44, 3445-50). Bevorzugt werden allerdings derartige Cyclen durch die Kondensation von α-Aminonitrilen oder α-Aminosäureamiden mit Aldehyden hergestellt. Die benötigten α-Aminonitrile können analog einer Streckersynthese gewonnen werden, die α-Aminosäureamide entstehen aus den entsprechenden α-Aminonitrilen durch Hydrolyse.

Die folgende Oxidation der Aminale kann nach für den Fachmann bekannten Methoden erfolgen. Vorteilhafterweise werden als Oxidationsmittel Halogene oder Halogenderivate eingesetzt. Als Halogene kommen für die Oxidation vorteilhafterweise die Verbindungen Chlor, Brom und Iod zum Einsatz. Geeignete Halogenderivate sind beispielsweise Hypochlorit, Chlorit, Dichloroxid, tert-Butylhypochlorit, Methylhypochlorit, Trichlorisocyanurat, Dichlorisocyanurat, Chloramin-T, N-Chlorsuccinimid, 1,3-Dichlor-5,5-dimethylhydantoin, 1-Chlor-3-brom-5,5-dimethylhydantoin, N-Chlorsuccinimid, Hypobromit, Bromat/Bromid, Bromchlorid, 1,3-Dibrom-5,5-dimethylhydantoin, 1-Chlor-3-brom-dimethylhydantoin, N-Bromsuccinimid, Methylhypobromit, u. a. Besonders bevorzugt ist elementares Chlor. Bevorzugt ist allerdings auch die Oxidation von Verbindungen der allgemeinen Formel II mit Mitteln aus der Gruppe der aktiven Sauerstoff enthaltenden Verbindungen. Als solche Substanzen werden u. a. angesehen Wasserstoffperoxid, Carosche Säure, etc. Ganz besonders bevorzugt ist der Einsatz von H₂O₂ oder dessen oxidativ wirkende Derivate, wie Na₂O₂, t-BuOOH, RCO₃H etc.

Auch elektrochemische Oxidations-Verfahren, wie sie beispielsweise aus Tetrahedron 1989, 45, 1691ff bekannt sind, lassen sich zur Oxidation der entsprechenden cyclischen 4-Oxoaminale zu 4-Oxoamidinen heranziehen. Unter Mithilfe eines Mediators werden daher Stickstoffatome im Molekül bei Anlegen einer Spannung substituiert. Eine abschließende Eliminierung bringt die gewünschten Amidine hervor. Eine für diesen Zweck einsetzbare elektrochemische Zelle kann dem Stand der Technik entlehnt werden. Als Mediatoren kommen prinzipiell alle dem Fachmann geläufigen Mediatoren in Frage. Ganz besonders bevorzugt ist der Einsatz von Chloridionen.

Ebenfalls lassen sich zur Oxidation Mittel aus der Gruppe VIII der Edelmetalle wie z. B. Ru, Rh, Pd, Os, Ir, Pt einsetzen. Diese in substöchiometrischen Mengen bevorzugt auf gängigen Trägermaterialien (wie z. B. Kohlenstoff, Al₂O₃, SiO₂, Carbonaten o. Oxiden) einzusetzenden Metalle führen zu einer Dehydrierung der Verbindungen der allgemeinen Formel II. Ganz besonders bevorzugt wird Palladium zur Dehydrierung eingesetzt. Ähnliche Dehydrierungen von Aminen wurden schon von Murahashi et al. (J. Am. Chem. Soc. 1983, 105, 5002-5011) beschrieben.

Bevorzugt wird eine Verbindung oxidiert bzw. dehydriert, bei welcher n = 1, die Reste R¹ und R² über deren verbindendes Kohlenstoffatom zu einem 5-gliedrigen Carbocyclus verbunden sind und R³ bzw. R⁵ beide gleich H und R⁴ ein linearer Butylrest ist. Ein derartiges Molekül bildet die Vorstufe zur Herstellung des Angiotensin-II-antagonisten Irbesartan® (Drugs of the Future 1997, 22, 481-491).

Die Dehydrierung wird bevorzugt in einem organischen Lösungsmittel aus der Gruppe der Ketone, Ether, Ester, Carbonsäureamid (DMF, N-Methylpyrolidinon), Alkane, Cycloalkane, halogenierte Kohlenwasserstoffe oder Gemische derselben durchgeführt, besonders bevorzugt ist ein Lösungsmittel aus der Reihe der Ketone, ganz besonders bevorzugt ist MIBK.

Das Temperaturintervall der Reaktion beträgt 50 °C bis 250°C, bevorzugt 90 °C bis 180 °C, ganz besonders bevorzugt 100-140°C.

Das zur Dehydrierung benötigte Metall wird im Bereich von 0,1 Gew.-% bis 10 Gew.-%, bevorzugt 0,5 Gew.-% bis 7 Gew.-%, ganz besonders bevorzugt 3 Gew.-% auf die zu dehydrierende Verbindung eingesetzt. Zur Anwendung kommt das Metall auf Trägern, bevorzugt auf Aktivkohle.

Die Herstellung der Verbindungen der allgemeinen Formel I erfolgt vorteilhafterweise durch Kristallisation als Salz. Zur Salzbildung können prinzipiell alle dem Fachmann bekannten anorganischen und organischen Säuren, wie z. B. HCl, HBr, RSO₃H, HCO₂H, (HOOC)₂, HClO₄, H₂SO₄, HBF₄, H₃PO₄, CF₃CO₂4, p-TosOH zum Einsatz kommen. Bevorzugt ist die Kristallisation als Hydrochloride.

Das nachfolgende Schema beschreibt nochmals die Herstellung der cyclischen 4-Oxoamidine für die Verbindungen mit n = 1 und R³, R⁵ = H.

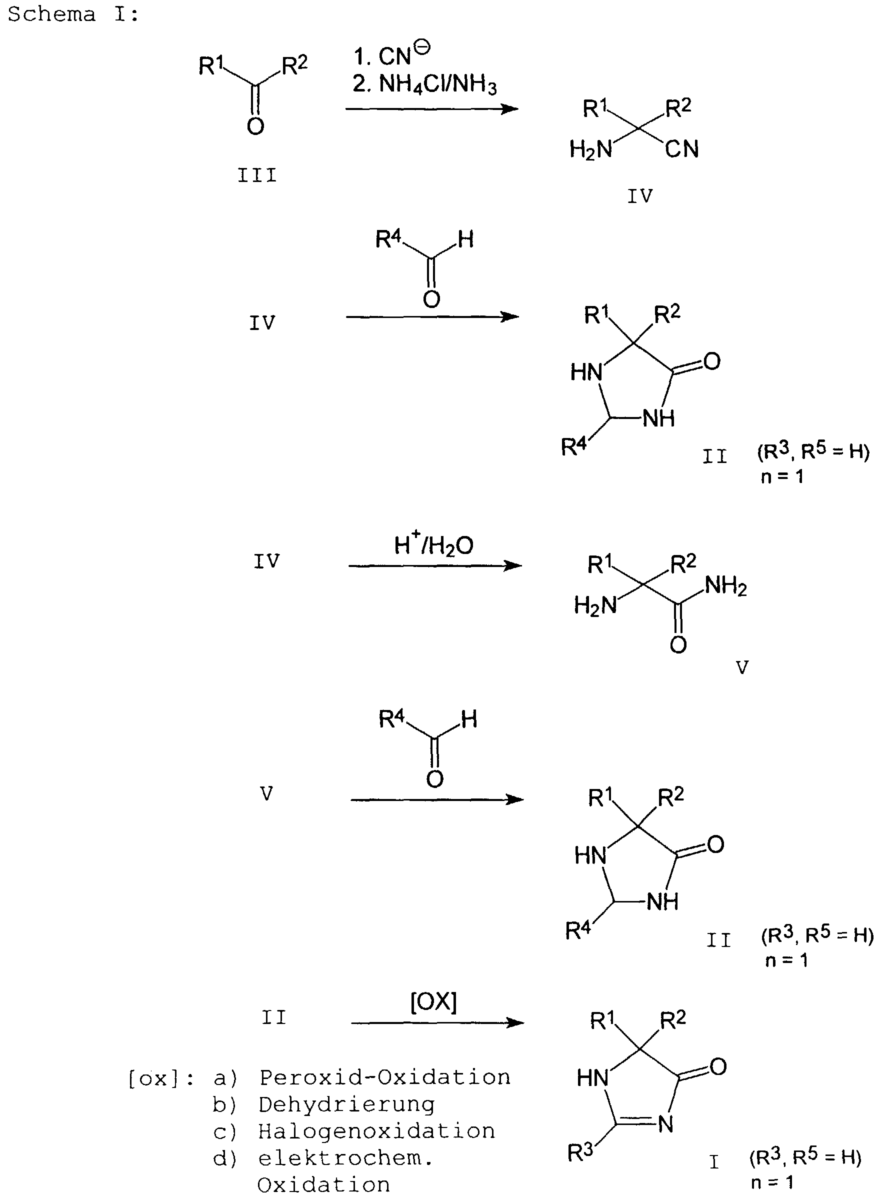

Zur Herstellung höher homologer Ringsysteme geht der Fachmann bevorzugt von β- oder γ-Aminonitrilen aus. Diese sind ebenfalls nach den dem Fachmann bekannten Methoden herzustellen (Lit.: J. prakt. Chem. 1984, 326, 279-86).

Als linear oder verzweigte (C₁-C₈)-Alkylreste sind anzusehen Methyl, Ethyl, n-Propyl, Isopropyl, n-Butyl, Isobutyl, sec-Butyl, tert.-Butyl, Pentyl, Hexyl, Heptyl oder Octyl samt aller ihrer Bindungsisomeren. Der linear oder verzweigte (C₂-C₈)-Alkenylrest umfaßt alle Substituenten, welche eben beim (C₁-C₈)-Alkylrest aufgezählt wurden mit Ausnahme vom Methylrest, wobei in diesen Resten mindestens eine Doppelbindung vorhanden ist. Der Umfang von (C₂-C₈)-Alkinyl entspricht dem von (C₂-C₈)-Alkenyl, wobei hier allerdings mindestens eine Dreifachbindung vorliegen muß. Der Rest (C₁-C₈)-Alkoxy entspricht dem Rest (C₁-C₈)-Alkyl mit der Maßgabe, daß dieser über ein Sauerstoffatom an den Cyclus gebunden ist. Als (C₂-C₈)-Alkoxyalkyl sind Reste gemeint, bei denen die Alkylkette durch mindestens eine Sauerstoffunktion unterbrochen ist, wobei nicht zwei Sauerstoffe miteinander verbunden sein können. Die Anzahl der Kohlenstoffatome gibt die Gesamtzahl der im Rest enthaltenen Kohlenstoffatome an. N-, O-, P-atomhaltige Reste sind insbesondere Alkyl, Alkenyl, Alkinyl-Reste der oben genannten Art, welche eines oder mehreren dieser Heteroatome in ihrer Kette aufweisen bzw. welche über eines dieser Heteroatome an das Amidin gebunden sind. Unter (C₃-C₇)-Cycloalkyl versteht man Cyclopropyl, Cyclobutyl, Cyclopentyl, Cyclohexyl bzw. Cycloheptylreste.

Die folgenden Beispiele sollen die Erfindung erläutern, sie jedoch keinesfalls einschränken.

### Beispiel 1a:

### Herstellung von 1-Aminocyclopentylnitril (ACCN):

Zu einer Lösung von 250 g NH₃ (25%ig; 3.67 mol) werden innerhalb von 45 min unter Kühlung bei 15 °C 193 g Cyclopentanoncyanhydrin (max. 1.74 mol) zugetropft. Das Reaktionsgemisch wird innerhalb von 45 min auf 50 °C erwärmt und 5.5 h bei dieser Temperatur gehalten. Das Gemisch wird auf 25-30 °C abgekühlt und anschließend mit 300 ml MTBE extrahiert. Die organische Phase wird abgetrennt und das Lösungsmittel bei 50 °C (25-30 mbar) abgedampft. Es werden 147 g 1-Aminocyclopentylnitril (90 %; GC) entsprechend einer Ausbeute von 69 % der Theorie erhalten.

### Beispiel 1b:

Zu einer Lösung von 45 g NH₄Cl in 200 g NH₃ (25%ig, 2.94 Mol) werden bei 35 °C innerhalb von 45 min 150 g Cyclopentanoncyanhydrin (max. 1.35 mol) zugegeben. Anschließend werden weitere 43 g NH₄Cl zugefügt und das Reaktionsgemisch 1.5 h bei 45 °C gerührt. Anschließend wird auf RT abgekühlt und die org. Phase abgetrennt. Es werden 120 g 1-Aminocyclopentylnitril (86 % Titration; 94 % GC) entsprechend einer Ausbeute von 69 % der Theorie erhalten.

### Beispiel 2:

### Herstellung von 1-Aminocyclopentylcarboxamid (ACCA):

Es werden 215 ml Schwefelsäure (96-98%ig) vorgelegt und auf 60 °C vorgewärmt. Dazu werden innerhalb von 60 min 125 g 1-Aminocyclopentannitril (85%ig; 0.965 mol) zudosiert. Das Reaktionsgemisch wird 60 min bei 60 °C und weitere 60 min bei 80 °C gerührt. Das Reaktionsgemisch wird bei 40-45 °C in 500 g NH₃ (25%ig) und 300 g H₂O zugegeben. Anschließend wird das Gemisch 6 x mit je 500 ml MIBK extrahiert und die vereinigten org. Phasen am Rotationsverdampfer weitestgehend eingeengt. Die Lösung wird auf 4 °C abgekühlt und das Kristallisat abfiltriert. Nach Trocknen werden 96 g 1-Aminocyclopentylcarboxamid (98.2 %; HPLC) entsprechend 76.2 % der Theorie erhalten.

### Beispiel 3:

### Herstellung von 2-Butyl-1,3-diaza-spiro[4.4]nonan-4-on (DSPC):

Eine Lösung von 126 g 1-Aminocyclopentancarboxamid (> 98 %, 0.98 Mol) in 150 g Wasser wird mit Ammoniak-Lösung (20 %; 20 g, 0.30 Mol) versetzt. Anschließend werden bei 15 °C 86 g Valeraldehyd (0.99 Mol) zugefügt. Hierbei steigt die Temperatur bis auf 32 °C an. Das Reaktionsgemisch wird auf 70 °C erhitzt und 7 h bei dieser Temperatur gerührt.
Zu der Emulsion werden 350 ml MIBK zugegeben und das Gemisch unter Rühren abgekühlt und die org. Phase abgetrennt. Der Extrakt wird weitgehend eingedampft (200 g) und mit 200 g n-Hexan versetzt und das Kristallisat abfiltriert. Nach Trocknung verbleiben 125 g 2-Butyl-1,3-diaza-spiro[4.4]nonan-4-on (HPLC: 98.6 %).
Die Hexanphase wird auf ca. 2/3 eingeengt und auf 15 °C abgekühlt. Es werden weitere 22 g Produkt isoliert entsprechend einer Ausbeute von 73 % der Theorie.
- ¹H-NMR (500 MHz, DMSO-d₆):: δ = 0.90 ppm (t, 3-H), 1.20-1.70 (m, 14-H), 1.80-1.95 (m, 1-H), 4.30 (t, 1-H), 8.20 (s, 1-H).
- ¹³C-NMR (125 MHz, DMSO-d₆):: δ = 18.8 ppm, 22.0, 24.7, 26.4, 36.0, 36.2, 36.9, 67.5, 68.1, 179.9.

M⁺ = 197

### Beispiel 4:

### Herstellung von 2-Butyl-1,3-diaza-spiro[4.4]nonan-4-on (DSPC):

Zu einer Lösung von 245 g einer Lösung von NH₃ (17.8 Gew.-%, 2.56 mol) mit 10 Gew.-% ACCN (24.6 g; 0.223 Mol) werden 100 g Cyclopentanoncyanhydrin (max. 0.90 mol) bei einer Temperatur von 35 °C unter Einleiten von NH₃ zugefügt. Das Gemisch wird 1 h bei 40 °C gerührt und anschließend die Wasserphase (264 g) abgetrennt. Die organische Phase wird in 200 ml MeOH gelöst und bei 10-20 °C mit 1.6 ml NaOH (50%ig) versetzt. Anschließend werden 90 g Valeraldehyd (1.03 mol) und 2 x 0.8 ml NaOH (50%ig) zugegeben, wobei die Temperatur auf 10-20 °C gehalten wird. Anschließend wird die Temperatur auf 60 °C erhöht und das Gemisch 2 h bei dieser Temperatur gerührt. Es werden 352 g Reaktionsgemisch mit einem Gehalt von 40.2 Gew.-% (HPLC, 141.5 g, 0.721 mol) DSPC entsprechend einer Ausbeute von 80.2 % gegenüber Cyclopentanoncyanhydrin erhalten.

### Beispiel 5:

### Herstellung von 2-Butyl-1,3-diaza-spiro[4.4]nonen-4-on x HCl (SPC) (Dehydrierung mit Pd/C):

130 g eines Reaktionsgemisch mit 29.6 g 2-Butyl-1,3-diaza-spiro[4.4]nonan-4-on (0.151 mol) in Methanol werden mit 80 g MIBK verdünnt und bei 90 °C innerhalb von 30 min mit 47 g HCl in Isopropanol (15.4%ig, 0.204 mol) versetzt. Die Suspension wird auf 30 °C abgekühlt und filtriert und mit MIBK gewaschen. Der Filterkuchen wird in 100 g MIBK suspendiert und bei 90 °C mit 14 ml NH₃-Lösung (25%ig; 0.166 mol) auf pH 7.5 eingestellt.
Die Wasserphase wird abgetrennt und die org. Phase azeotrop entwässert (7 g Destillat).

Die MIBK-Lösung wird mit 10 g Pd/C versetzt und 12 h unter Rückfluß erhitzt. Der Katalysator wird warm abfiltriert und das Filtrat bei 90 °C mit einer Lösung von 47 g HCl in Isopropanol (15.4 %; 0.204 mol) versetzt. Die Suspension wird auf 25 °C abgekühlt, mit ca. 40 g MIBK gewaschen und getrocknet. Es werden 26 g (99 %; HPLC; >95 % NMR) entsprechend einer Ausbeute von 74 % der Theorie erhalten.
- ¹H-NMR (500 MHz, DMSO-d₆):: δ = 0.90 ppm (t, 3-H), 1.32 (sex, 2-H), 1.65-2.05 (m, 10-H), 2.78 (t, 2-H), 13.1-14.0 (m, 2-H).
- ¹³C-NMR (125 MHz, DMSO-d₆):: δ = 13.28 ppm, 21.35, 24.88, 26.54, 27.23, 36.43, 72.03, 173.18, 179.49.

### Beispiel 6:

### Herstellung von 2-Butyl-1,3-diaza-spiro[4.4]nonen-4-on x HCl (SPC) (Oxidation mit Chlor):

a) Eine Lösung von 36 g DSPC (0.18 mol) in 128 g MeOH wird mit 22 g Na₂CO₃ (0.21 mol) vermischt und bei einer Temperatur von 5-10 °C mit 30 g Chlor (0.42 mol) versetzt. Anschließend wird 1 h bei 10-15 °C nachgerührt und die Suspension filtriert. Das Filtrat wird 2 h bei 60 °C erhitzt und anschließend auf 20 °C abgekühlt. Der entstandene Feststoff wird abfiltriert und getrocknet. Es werden 11 g SPC (94.6 % HPLC) erhalten. Die Mutterlauge wird weitgehend eingeengt und der Rückstand mit 150 g MIBK aufgerührt. Nach Filtration und Trocknung werden weitere 15 g SPC x HCl (93.5 % HPLC) entsprechend einer Ausbeute von 51 % der Theorie erhalten.
b) 176 g einer DSPC Reaktionslösung (46.1 Gew.-%, 0.41 mol) wird im Vakuum bei 70 °C (20 mbar) eingeengt. Der Rückstand wird in 324 g MeOH gelöst und mit 45 g Na₂CO₃ (0.42 mol) vermischt. Anschließend werden bei 10 °C insgesamt 79 g Chlor (1.11 mol) eingeleitet und das Reaktionsgemisch wird 1 h bei 20 °C nachgerührt. Die Suspension wird vom Feststoff abfiltriert und das Filtrat wird bei 60 °C 1 h erhitzt. Anschließend wird MeOH unter Vakuum abdestilliert und 600 g MIBK zugegeben. Methanol wird durch Destillieren bis 115 °C vollständig abdestilliert und der ausgefallene Feststoff abfiltriert und getrocknet. Es werden 83 g SPC x HCl (91 % HPLC) gemäß einer Ausbeute von 79.5 % der Theorie erhalten.
C) 150 g einer DSPC Reaktionslösung (35.4 %, 53 g, 0.27 mol) werden im Vakuum bei 70 °C (20 mbar) eingeengt und der Rückstand in 220 g MeOH aufgenommen. Es werden 50 g Na₂CO₃ (0.47 mol) zugegeben und bei einer Temperatur von 10 °C 75 g Chlor (1.05 mol) eingeleitet. Die Suspension wird filtriert und das Filtrat auf 60 °C erhitzt. Es werden weitere 125 g MeOH zugegeben, und das Reaktionsgemisch wird 3 h bei dieser Temperatur gerührt. Das Lösungsmittel wird weitestgehend abdestilliert und der Rückstand mit 500 g MIBK versetzt. Die Suspension wird von Resten MeOH befreit (T = 115 °C) und der Feststoff abfiltriert. Nach Trocknen wurden 53 g SPC x HCl (89 % HPLC) entsprechend einer Ausbeute von 76 % der Theorie erhalten.

Es werden weiterhin Reaktionen bei höherer Temperatur durchgeführt, um den ersten und den zweiten Schritt gleichzeitig durchzuführen.

### Beispiel 7:

### Herstellung von 2-Butyl-1,3-diaza-spiro[4.4]nonen-4-on x HCl (SPC) (Vollständiges Durchlaufen des Prozesses):

Zu 475 g Cyclopentanon (5.64 mol) werden 1.4 ml Triethylamin zugegeben und das Gemisch auf 20 °C gekühlt. Dazu wird unter Kühlung innerhalb von 1 h 152.5 g HCN (5.64 mol) so dosiert, daß die Reaktionstemperatur < 20 °C beträgt. Das Reaktionsgemisch wird weitere 60 min bei 30 °C gerührt und auf Raumtemperatur abgekühlt. Anschließend wird durch Zugabe von 0.5 ml H₂SO₄ (96-98 %) auf pH 1 eingestellt. Dieses Cyanhydrin wird aliquotiert für die weiteren Umsetzungen eingesetzt.

Zu einer Lösung von 240 g Aminierungswasserphase werden 5 g NH₄Cl zugegeben. Dazu werden unter Einleiten von NH₃ 100 g Cyclopentanoncyanhydrin (max. 0.90 mol) zugegeben, wobei die Temperatur auf 35 °C ansteigt. Nach 90 min bei 40 °C wird die untere wäßrige Phase abgetrennt.
Es werden 200 ml Methanol und 1.6 ml NaOH (50 %) vorgelegt und dazu gleichzeitig die org. Phase und 90 g Valeraldehyd (1.03 mol) so zudosiert, daß die Reaktionstemperatur bei 10-20 °C gehalten wird. Nach Dosieren von 45 g und 90 g Valeraldehyd werden weitere 0.8 ml NaOH (50%ig) zugegeben. Anschließend wird das Reaktionsgemisch 2 h bei 60 °C erhitzt. Es werden 357 g einer Lösung mit 41.8 Gew.-% 2-Butyl-1,3-diaza-spiro[4.4]nonan-4-on (HPLC, 149.2 g, 0.760 mol) erhalten. Die Lösung wurde eingeengt (70 °C, 25 mbar) und der Rückstand in 600 g MIBK gelöst.
Eine Lösung von 99.5 g DSPC (max. 0.507 mol) in 400 g MIBK werden bei 85 °C mit 100 g einer Lösung von HCl in Isopropanol (25.5 %, 0.70 mol) und mit 150 g Isopropanol verdünnt. Der Niederschlag wird abfiltriert und mit MIBK gewaschen.
Der Filterkuchen wird in 720 g MIBK suspendiert und bei 85 °C mit 100 ml H₂O und 56 g NaOH (50%ig, 0.70 mol) ausgerührt. Die wäßrige Phase wird abgetrennt und organische Phase azeotrop entwässert. Es wurden 327 g Destillat bis 115 °C abgenommen. Anschließend werden 48 g Katalysator zugegeben und die Reaktionsmischung 8 h unter Rückfluß erhitzt. Der Katalysator wurde abfiltriert und das Filtrat mit 85 g HCl (32%ig, 0.75 mol) von pH 7 auf pH 0 gebracht.
Die Suspension wird nun bis zu einer Kopftemperatur von 114 °C azeotrop entwässert (75 g H₂O). Die Suspension wird filtriert und der Filterkuchen getrocknet. Es werden 107 g (>95 % NMR, 0.440 mol) 2-Butyl-1,3-diaza-spiro[4.4]nonan-4-on x HCl entsprechend einer Ausbeute von 73 % bezogen auf Cyclopentanoncyanhydrin erhalten.

### Beispiel 8:

### Herstellung von 2-Butyl-4-methyl-4-(2-methylethyl)-imidazolidin-5-on:

20.0 g 2-Amino-2,3-dimethylbutyramid (> 96 %, 0.147 mol) werden bei RT in 100 ml H₂O und 50 ml Methanol gelöst und mit 13.2 g Valeraldehyd (0.152 mol) vermischt. Das Gemisch wird 2.5 h auf 70 °C erhitzt. Das zweiphasige Gemisch wird 3 x mit je 200 ml MIBK bei Raumtemperatur extrahiert und die vereinigten org. Phasen im Vakuum eingeengt. Es wurden 25.3 g (97.8 % (cis/trans-Gemisch: 25:72; keine Zuordnung) eines weißen Feststoffs entsprechend einer Ausbeute von 82 % der Theorie erhalten.

### Beispiel 9:

### Herstellung von 2-Butyl-4-methyl-4-(methylethyl)-1H-imidazol-5(4H)-on x HCl

Eine 20%ige Lösung von 2-Butyl-4-methyl-4-(2-methylethyl)-imidazolidin-5-on in MIBK wird mit Pd/C vermischt und 12 h unter Rückfluß erhitzt. Anschließend wird der Katalysator abfiltriert und das Filtrat mit HCl in Isopropanol umgesetzt. Der entstandene Feststoff wird abfiltriert und getrocknet (Reinheit > 95 % NMR).
- ¹H-NMR (500 MHz, DMSO-d₆):: δ = 0.82 ppm (d, 3-H), 0.92 (t, 3-H), 1.00 (d, 3-H) 1.35 (sex, 2-H), 1.42 (s, 3-H), 2.80-2.95 (m, 2-H,13.2-14.0 (m, 2-H).

### Beispiel 10:

### Herstellung von 2-Butyl-1,3-diaza-spiro[4.4]nonen-4-on (SPC) (Oxidation der Reaktionslösung mit Chlor)

176 g einer DSPC Reaktionslösung (46.1 Gew.-%; 0.41 mol) wird im Vakuum bei 70 °C (20 mbar) eingeengt. Der Rückstand wird in 324 g MeOH gelöst und mit 45 g Na₂CO₃ (0.42 mol) vermischt. Anschließend werden bei 10 °C insgesamt 79 g Chlor (1.11 mol) eingeleitet, und das Reaktionsgemisch wird 1 h bei 20 °C nachgerührt. Die Suspension wird vom Feststoff abfiltriert, und das Filtrat wird bei 60 °C 1 h erhitzt. Anschließend wird MeOH unter Vakuum abdestilliert und 600 g MIBK zugegeben. Methanol wird durch Destillieren bis 115 °C vollständig abdestilliert und der ausgefallene Feststoff abfiltriert und getrocknet. Es werden 83 g SPC (91 % HPLC) gemäß einer Ausbeute von 79.5 % der Theorie erhalten.

## Patentansprüche

1. Verfahren zur Herstellung von cyclischen 4-Oxoamidinen der allgemeinen Formel I 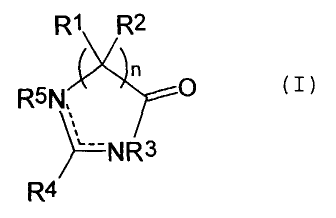 oder deren Salze
worin
R¹, R² für gleiche oder unterschiedliche Indices n unabhängig voneinander stehen für
H, (C₁-C₈)-Alkyl, (C₂-C₈)-Alkenyl, (C₂-C₈)-Alkinyl, (C₁-C₈)-Alkoxy, (C₂-C₈)-Alkoxyalkyl, welche gegebenenfalls linear oder verzweigt vorliegen sowie einfach oder mehrfach mit Halogenen, N-, O-, P-atomhaltigen Resten substituiert sein können,
(C₃-C₇)-Cycloalkyl, welche gesättigt und/oder ungesättigt sowie einfach oder mehrfach mit linear oder verzweigten (C₁-C₈)-Alkyl, (C₂-C₈)-Alkenyl, (C₁-C₈)-Alkoxy, (C₂-C₈)-Alkoxyalkyl, Halogenen, N-, O-, P-atomhaltigen Resten substituiert sein oder im Ring Heteroatome wie N, O, P enthalten können,
Aryl, wie Phenyl und/oder Naphthyl, Aralkyl, wie Benzyl und/oder Phenethyl,
Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl,
wobei die eben genannten Cyclen gegebenenfalls einfach oder mehrfach mit linear oder verzweigten (C₁-C₈)-Alkyl, (C₂-C₈)-Alkenyl, (C₁-C₈)-Alkoxy, (C₂-C₈)-Alkoxyalkyl, Halogenen, N-, O-, P-atomhaltigen Resten substituiert sein können
oder
R¹ und R² über das verbindende Kohlenstoffatom zu einem 3- bis 7-gliedrigen carbocyclischen oder heterocyclischen, gesättigten oder ungesättigten Ring verbunden sein können, welcher gegebenenfalls einfach oder mehrfach mit linear oder verzweigten (C₁-C₈)-Alkyl, (C₂-C₈)-Alkenyl, (C₁-C₈)-Alkoxy, (C₂-C₈)-Alkoxyalkyl, Halogenen, N-, O-, P-atomhaltigen Resten substituiert sein kann,
R³, R⁴, R⁵ unabhängig voneinander die Bedeutung der Reste für R¹ annehmen können, mit der Maßgabe, daß R³ und R⁵ nicht gleichzeitig ungleich H sein dürfen und die Reste der Gruppe R³, R⁴ oder R⁵ untereinander nicht zu einem Cyclus verbunden sind,
n eine ganze Zahl zwischen 1 und 3 ist,
**dadurch gekennzeichnet,**
daß ein Cyclus der allgemeinen Formel II 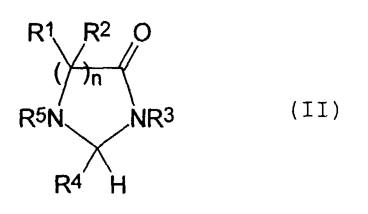 oder dessen Salz zu Verbindungen der Formel I oxidiert bzw. dehydriert wird.

2. Verfahren nach Anspruch 1,
**dadurch gekennzeichnet,**
daß man Verbindungen der allgemeinen Formel II durch Umsetzung von α-Aminonitrilen mit Aldehyden
oder durch Umsetzung von α-Aminosäureamiden mit Aldehyden herstellt.

3. Verfahren nach Anspruch 1,
**dadurch gekennzeichnet,**
daß man die Oxidation mit einem Mittel aus der Gruppe der Halogene oder Halogenderivate durchführt.

4. Verfahren nach Anspruch 3,
**dadurch gekennzeichnet,**
daß man als Oxidationsmittel Cl₂ verwendet.

5. Verfahren nach Anspruch 1,
**dadurch gekennzeichnet,**
daß man zur Oxidation ein Mittel aus der Gruppe der aktiven Sauerstoff enthaltenden Verbindungen verwendet.

6. Verfahren nach Anspruch 5,
**dadurch gekennzeichnet,**
daß man H₂O₂ oder deren oxidativ wirkende Derivate verwendet.

7. Verfahren nach Anspruch 1,
**dadurch gekennzeichnet,**
daß man die Oxidation elektrochemisch durchführt.

8. Verfahren nach Anspruch 7,
**dadurch gekennzeichnet,**
daß man als Mediator Chloridionen einsetzt.

9. Verfahren nach Anspruch 1,
**dadurch gekennzeichnet,**
daß man zur Dehydrierung ein Mittel aus der Gruppe Ru, Rh, Pd, Os, Ir, Pt einsetzt.

10. Verfahren nach Anspruch 9,
**dadurch gekennzeichnet,**
daß man die Verbindung der allgemeinen Formel II in Gegenwart von Palladium dehydriert.

11. Verfahren nach Anspruch 10, **dadurch gekennzeichnet,**
daß man Verbindungen der allgemeinen Formel II oxidiert bzw. dehydriert,
worin
R¹, R² über das verbindende Kohlenstoffatom zu einem 5-gliedrigen Carbocyclus verbunden sind,
R³, R⁵ beide gleich H sind und
R⁴ ein linearer Butylrest ist.

12. Verfahren nach Anspruch 9,
**dadurch gekennzeichnet,**
daß man die Dehydrierung in einem organischen Lösungsmittel oder Lösungsmittelgemisch durchführt.

13. Verfahren nach Anspruch 12,
**dadurch gekennzeichnet,**
daß man in einem Keton dehydriert.

14. Verfahren nach Anspruch 13,
**dadurch gekennzeichnet,**
daß man in MIBK dehydriert.

15. Verfahren nach Anspruch 14,
**dadurch gekennzeichnet,**
daß man in einem Temperaturintervall von 50 °C bis 250 °C arbeitet.

16. Verfahren nach Anspruch 15,
**dadurch gekennzeichnet,**
daß man in einem Temperaturintervall von 90 °C bis 180 °C arbeitet.

17. Verfahren nach Anspruch 9,
**dadurch gekennzeichnet,**
daß man zwischen 0,1 Gew.-% und 10 Gew.-% an Metall bezogen auf die zu dehydrierende Verbindung einsetzt.

18. Verfahren nach Anspruch 17,
**dadurch gekennzeichnet,**
daß man zwischen 5 Gew.-% und 7 Gew.-% an Katalysator einsetzt.

19. Verfahren nach Anspruch 1,
**dadurch gekennzeichnet,**
daß man das cyclische 4-Oxoamidin als dessen Hydrochlorid herstellt.
